(19) Europäisches Patentamt
European Patent Office
Office européen des brevets

(11) Publication number: **0 257 351 A2**

(12) # EUROPEAN PATENT APPLICATION

(21) Application number: 87111130.8

(51) Int. Cl.[4]: C07D 413/04 , A61K 31/42

(22) Date of filing: 31.07.87

(30) Priority: 27.08.86 US 900857
10.04.87 US 36831
10.04.87 US 36838

(43) Date of publication of application:
02.03.88 Bulletin 88/09

(84) Designated Contracting States:
AT BE CH DE ES FR GB IT LI LU NL SE

(71) Applicant: **PENNWALT CORPORATION**
**Pennwalt Building Three Parkway**
**Philadelphia Pennsylvania 19102(US)**

(72) Inventor: **Georgiev, Vassil Stefanov**
**P.O. Box 1032**
**Penfield New York 14526(US)**
Inventor: **Mullen, George Byron**
**1385 Jenks Road**
**Avon New York 14414(US)**

(74) Representative: **Kraus, Walter, Dr. et al**
**Patentanwälte Kraus, Weisert & Partner**
**Thomas-Wimmer-Ring 15**
**D-8000 München 22(DE)**

(54) **3-Phenyl-3-(1H-1,2,4-triazol-1-ylmethyl)-5-substituted-2-methyl-isoxazolidines.**

(57) 3-phenyl-3-(1H-1,2,4-triazol-1-ylmethyl)-5-substituted-2-methylisoxazolidines are useful as antifungal agents.

EP 0 257 351 A2

## 3-Phenyl-3-(1H-1,2,4-triazol-1-ylmethyl)-5-substituted-2-methylisoxazolidines IR 28671 (Combination of IR 2867, IR 2893 and IR 2897)

### Background of the Invention

This invention relates generally to substituted 2-methylisoxazolidines and more specifically to 3-phenyl-3-(1H-1,2,4-triazol-1-ylmethyl)-5-substituted-2-methylisoxazolidines which are useful as antifungal agents.

### Brief Summary of the Invention

In accordance with this invention there are provided compounds of the formula:

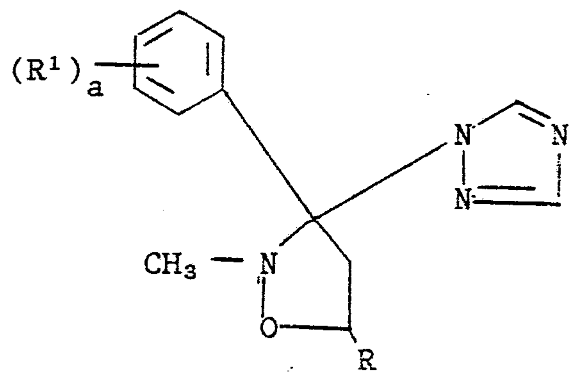

wherein;
a = 1 or 2,
$R^1$ is selected from hydrogen, lower alkyl, lower alkoxy, halogen and combinations thereof provided that the ortho position is hydrogen, and
R is selected from,

(a) 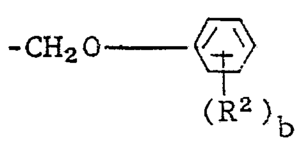

wherein;
b = 1 or 2 and
$R^2$ is selected from hydrogen, lower alkyl, lower alkoxy halogen, alkoxycarbonyl, alkanoylamino, nitro, and combinations thereof,
(b) $-(CH_2)_nCH_3$
wherein,
n = 1 to 18, branched or unbranched chain, and

(c) 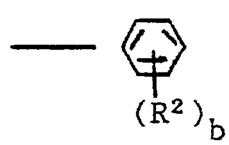

wherein,
b = 1 or 2, and
$R^2$ is selected from hydrogen, lower alkyl, lower alkoxy, halogen, trifluoromethyl, nitro, and combinations



BAD ORIGINAL

thereof,
and pharmaceutically acceptable acid addition salts thereof, in the form of their enantiomers, or mixtures of their enantiomers including diastereoisomeric pairs of such enantiomers.

## Detailed Description of the Invention

The compounds of this invention are useful as antifungal agents. They have in vitro activity against yeast and systemic mycoses and dermatophytes as determined by broth and agar testing techniques [-(McGinnis, M.R., Laboratory Handbook of Medical Mycology, Academic Press, N.Y., N.Y. (1980)]. The compounds have good to moderate inhibitory activity (minimum inhibitory concentrations, MIC, of <.2 to 70 ug/ml) against a variety of organisms including epidermophyton floccosum, trichophyton rubrum, trichophyton schoenleinii, candida stellatoidea, microsporum audouini and microsporum canis.

Because of the antifungal activities of the compounds of the invention they can be used, for example, in suitable liquid, semi-solid or solid carriers in the form of solutions, emulsions, suspensions, dispersions, ointments, aerosols, soaps, detergents, and powders in amounts effective to combat systemic and dermatophylic fungal infections in warm blooded animals (1 to 20 percent active ingredient).

In the definitions of the compounds of the invention by halogen is meant chlorine, bromine, fluorine and iodine with chlorine and fluorine being preferred. By lower alkyl is meant $C_1$ to $C_4$ and by lower alkoxy is meant $C_1$ to $C_6$ which, in either case, can have a branched or unbranched chain. Compounds having ortho substitution of the 3-phenyl group were not prepared probably due to steric hindrance.

The 3-phenyl-3-(1H-1,2,4-triazol-1-ylmethyl)-2-methyl-5-substituted isoxazolidine derivatives are obtained as mixtures of cis-and trans-diastereomers due to the presence in the isoxazolidine ring of two asymmetric carbon atoms. The diastereomeric mixture is conveniently separated by flash-chromatography on silica gel using halogenated hydrocarbons (preferably methylene chloride and chloroform), alkanols (preferably methanol and ethanol), and ethyl acetate as eluents. The eluents may be used alone or in combinations such as the ones comprised of 95-99% halogenated hydrocarbon and 1-5% alkanol by volume. The stereochemistry of the two asymmetric carbon atoms in the isoxazolidine ring may be determined by conventional methods that include X-ray crystallography, nuclear magnetic resonance spectroscopy, circular dichroism and optical rotatory dispersion. Both the cis- and trans-diastereoisomers are resolvable into their optical enantiomers with (+)-and (-)-optical rotations by standard techniques such as fractional recrystallization of the diastereomeric salts with optically active organic acids such as (+)-and (-)-tartaric acid, (+)-and (-)-dibenzoyltartaric acid and the like.

The compounds can be prepared as illustrated in the following diagrams. The synthesis of the nitrone precursors 1 is accomplished by reacting an appropriately substituted triazolylacetophenone compound with N-methylhydroxylamine hydrochloride in absolute ethanol at room or elevated (70-75°C) temperatures in the presence of base, for example alkali metal carbonates, bicarbonates or acetates under a nitrogen atmosphere. The triazolylacetophenones are prepared according to known procedures, for example, Godefroi et al., J. Medicinal Chem. 12, 784 (1969) and Nardi et al., J. Medicinal Chem. 24 , 727 (1981).

Reaction of the N-methyl-1-phenyl-2-(1H-1,2,4-triazol-1-yl) ethanimine N-oxide nitrone derivative 1 with an allyl (substituted)phenyl ether (2 ) provides a diastereomeric mixture of the desired cis-and trans-3-(substituted phenyl)-3-(1H-1,2,4-triazol-1-ylmethyl)-2-methyl-5-[(substituted phenoxy)methyl]isoxazolidine derivative 3.

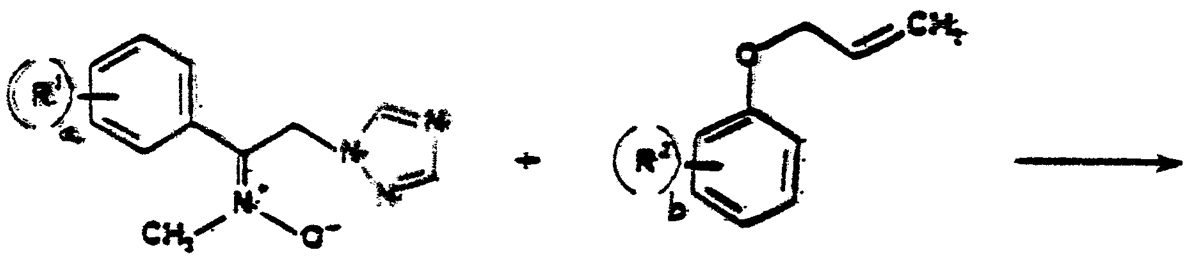

1 2

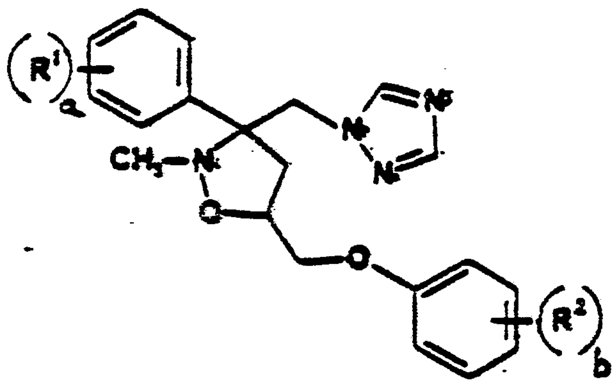

3

Similarly, other compounds of the invention are prepared by the reaction of the nitrone precursors with: (a) 1-alkenes 4 having 4 to 21 carbons to give compounds 5; or

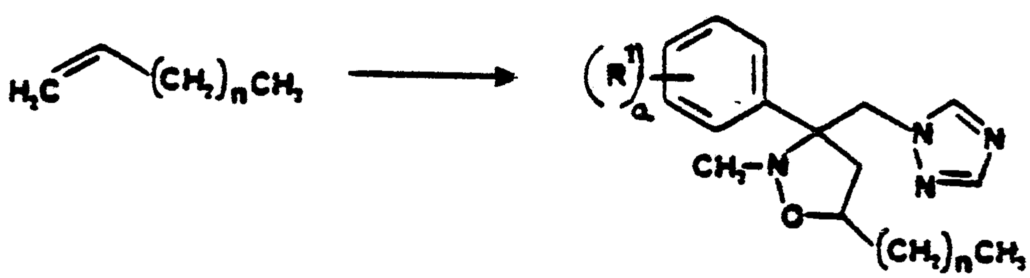

4 5

(b) styrene derivatives 6 to give compounds 7.

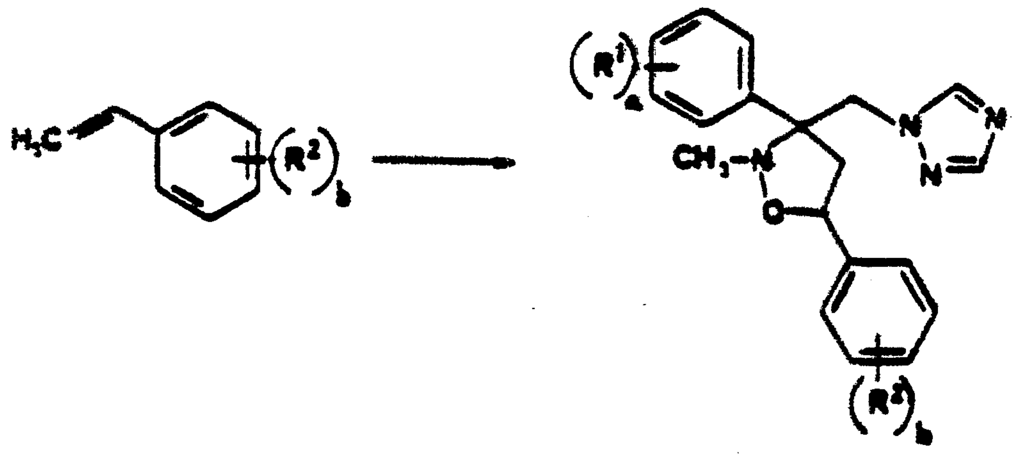

6 7

## Preparation of Styrene Compounds

The styrene derivatives 6 are either commercially available or may be prepared by standard procedures such as a Wittig reaction of an appropriately substituted benzaldehyde with methylenetriphenylphosphorane [Wittig and Schoellkopf, Chem. Ber. 87 , 1318 (1954)].

The compounds of the invention are all basic and thus can form salts with pharmaceutically acceptable inorganic and organic acids such as, for example, acetic acid, maleic acid, malic acid, fumaric acid, succinic acid, succinamic acid, tartaric acid, citric acid, lactic acid, hydrochloric acid, hydrobromic acid, hydroiodic acid, nitric acid, sulfuric acid and phosphoric acid.

The preparation of the compounds of the invention is further illustrated by the following examples.

### Example 1

3-(3-Methylphenyl)-3-(1H-1,2,4-triazol-1-ylmethyl)-2-methyl -5-[(2-nitrophenoxy)methyl]isoxazolidine (3,$R^1$ = 3-$CH_3$, $R^2$ = 2-$NO_2$)

A solution of 8.0 g (35 mmol) of N-methyl-1-(3-methylphenyl) -2-(1H-1,2,4-triazol-1-yl)ethanimine N-oxide (1, $R^1$ = 3-$CH_3$) [prepared by reacting 2-(1H-1,2,4-triazol-1-yl)-3′-methylacetophenone (7.04 g, 0.035 mol) with N-methylhydroxylamine hydrochloride (3.51 g, 0.042 mol) and sodium bicarbonate (3.53 g, 0.042 mol) in 100 ml of ethanol] and 9.34 g (52 mmol) of allyl 3-methylphenyl ether (2, $R^2$ = 2-$NO_2$) in 200 ml of toluene was refluxed under nitrogen atmosphere for 65 hours. Upon cooling to room temperature, the solvent was removed under reduced pressure leaving a dark oil which was dissolved in ethyl acetate. Addition of ether caused crystallization to occur, yielding 8.94 g (62%) of compound 3 ($R^1$ = 3-$CH_3$, $R^2$ = 2-$NO_2$). The resulting cis-and trans-diastereomeric mixture of the title compound was flash-chromatographed on neutral silica gel using chloroform-methanol (99:1) by volume as eluent.

Isomer A has a melting point of 149-152°C (ethyl acetate).

Anal. Calcd for $C_{21}H_{23}N_5O_4$: C, 61.60; H, 5.66; N, 17.10. Found: C, 61.27; H, 5.93; N, 16.94.

Example 2

3-Phenyl-3-(1H-1,2,4-triazol-1-ylmethyl)-2-methyl-5-(phenoxymethyl)isoxazolidine (3, $R^1 = R^2 = H$)

Compound 3 ($R^1 = R^2 = H$) was prepared by a procedure similar to that described in Example 1 by reacting N-methyl-1-phenyl-2-(1H-1,2,4-triazol-1-yl)ethanimine N-oxide (1, $R^1 = H$) with allyl phenyl ether (2, $R^2 = H$). The resulting cis-and trans-diastereomeric mixture of the title compound was flash-chromatographed on neutral silica gel using chloroform-methanol (99:1 by volume) as eluent. Isomer A has a melting point 68-75°C (ethyl acetate-hexane, 1:1 by volume).
Anal. Calcd for $C_{20}H_{22}N_4O_2$: C, 68.55; H, 6.33; N, 15.99. Found: C, 68.70; H, 6.44; N, 15.95.

Example 3

3-Phenyl-3-(1H-1,2,4-triazol-1-ylmethyl)-2-methyl-5-[(4-chlorophenoxy)methyl]isoxazolidine (3, $R^1 = H$, $R^2 = 4$-Cl)

Compound 3 ($R^1 = H$, $R^2 = 4$-Cl) was prepared by a procedure similar to that described in Example 1 by reacting N-methyl-1-phenyl-2-(1H-1,2,4-triazol-1-yl)ethanimine N-oxide ( 1, $R^1 = H$) with allyl 4-chlorophenyl ether (2, $R^2 = 4$-Cl). The resulting cis-and trans-diastereomeric mixture of compound 3 ($R^1 = H$, $R^2 = 4$-Cl) was flash-chromatographed on neutral silica gel using chloroform-methanol (98:2 by volume) as eluent. Isomer A has a melting point of 144-147°C (ethyl acetate).
Anal. Calcd for $C_{20}N_{21}ClN_4O_2$: C, 62.42; H, 5.50; N, 14.56; Cl, 9.21. Found: C, 62,40; H, 5.54; N, 14.54; Cl, 9.14.

Example 4

3-Phenyl-3-(1H-1,2,4-triazol-1-ylmethyl)-2-methyl-5-[(4-fluorophenoxy)methyl]isoxazolidine (3, $R^1 = H$, $R^2 = 4$-F)

Compound 3 ($R^1 = H$, $R^2 = 4$-F) was prepared by a procedure similar to that described in Example 1 by reacting N-methyl-1-phenyl-2-(1H-1,2,4-triazol-1-yl)ethanimine N-oxide (1, $R^1 = H$) with allyl 4-fluorophenyl ether (2, $R^2 = 4$-F). The resulting cis-and trans-diastereomeric mixture of derivative 3 ($R^1 = H$, $R^2 = 4$-F) was flash-chromatographed on neutral silica gel using chloroform-methanol (98:2 by volume) as eluent. Isomer A has a melting point of 115-120°C (ethyl acetate).
Anal. Calcd for $C_{20}H_{21}FN_4O_2$; C, 65.20; H, 5.75; N, 15.21; F, 5.16. Found: C, 65.13; H, 5.79; N, 15.13; F, 5.26.

Example 5

3-Phenyl-3-(1H-1,2,4-triazol-1-ylmethyl)-2-methyl-5-[(4-acetamidophenoxy)methyl]isoxazolidine (3, $R^1 = H$, $R^2 = 4$-$NHCOCH_3$)

Compound 3 ($R^1 = H, R^2 = 4$-$NHCOCH_3$) was prepared by a procedure similar to that described in Example 1 by reacting N-methyl-1-phenyl-2-(1H-1,2,4-triazol-1-yl)ethanimine N-oxide (1, $R^1 = H$) with 4-acetamidophenyl allyl ether (2 , $R^2 = 4$-$NHCOCH_3$). The resulting cis-and trans-diastereomeric mixture of compound 3 ($R^1 = H$, $R^2 = 4$-$NHCOCH_3$) was flash-chromatographed on neutral silica gel using chloroform-methanol (97:3 by volume) as eluent. Isomer has a melting point of 186-189°C (benzene-methanol, 1:1 by volume).
Anal. Calcd for $C_{22}H_{25}N_5O_3$: C, 64.85; H, 6.18; N, 17.19. Found: C, 64.80; H, 6.26; N, 17.35.



BAD ORIGINAL

Example 6

3-(4-Chlorophenyl)-3-(1H-1,2,4-triazol-1-ylmethyl)-2-methyl -5-(phenoxymethyl)isoxazolidine (3, $R^1$ = 4-Cl, $R^2$ = H)

Compound 3 ($R^1$ = 4-Cl, $R^2$ = H) was prepared by a procedure similar to that described in Example 1 by reacting 1-(4-chlorophenyl)-N-methyl-2-(1H-1,2,4-triazol-1-yl)ethanimine N-oxide (1, $R^1$ = 4 = Cl) with allyl phenyl ether (2, $R^2$ = H).

The resulting cis-and trans-diastereomeric mixture of compound 3 ($R^1$ = 4-Cl, $R^2$ = H) was flash-chromatographed on neutral silica gel using chloroform-methanol (98:2 by volume) as eluent.
Isomer A has a melting point of 135-139°C (ethyl acetate-hexane, 1:1 by volume)
Anal. Calcd for $C_{20}H_{21}ClN_4O_2$: C, 62.42; H, 5.50; N, 14.56; Cl, 9.21. Found: C, 62.37; H, 5.64; N, 14.47; Cl, 9.54. Isomer B has a melting point of 97-100°C (ethyl acetate-hexane, 1:1 by volume).
Anal. Calcd for $C_{20}N_{21}ClN_4O_2$: C, 62.42; H, 5.50; N, 14.56; Cl, 9.21. Found: C, 61.68; H, 5.51; N, 14.40; Cl, 10.21.

Example 7

3-(4-Chlorophenyl)-3-(1H-1,2,4-triazol-1-ylmethyl)-2-methyl -5-[(4-chlorophenoxy)methyl]isoxazolidine (3, $R^1$ = $R^2$ = 4 = Cl)

Compound 3 ($R^1$ = $R^2$ = 4-Cl) was prepared by a procedure similar to that described in Example 1 by reacting 1-(4-chlorophenyl)-N-methyl-2-(1H-1,2,4-triazol-1-yl)ethanimine N-oxide (1, $R^1$ = 4-Cl) with allyl 4-chlorophenyl ether (2, $R^2$ = 4-Cl). The resulting cis-and trans-diastereomeric mixture of the title compound was purified by high-pressure liquid chromatography on neutral silica gel using chloroform-methanol (98:2 by volume) as eluent. Isomer A has a melting point of 125-129°C (ethyl acetate).
Anal. Calcd for $C_{20}H_{20}Cl_2N_4O_2$: C, 57.29; H, 4.81; N, 13.36; Cl, 16.91. Found: C, 57.30; H, 4.89; N, 13.37; Cl, 16.74.

The above preparation was repeated except that nitrone 1 was prepared using sodium acetate in place of sodium bicarbonate.

Example 8

3-(4-Chlorophenyl)-3-(1H-1,2,4-triazol-1-ylmethyl)-2-methyl-5-[(4-methoxyphenoxy)methyl]isoxazolidine (3, $R^1$ = 4-Cl, $R^2$ = 4-$OCH_3$)

Compound 3 ($R_1$ = 4-Cl, $R^2$ = 4-$OCH_3$) was prepared by a procedure similar to that described in Example 1 by reacting 1-(4-chlorophenyl)-N-methyl-2-(1H-1,2,4-triazol-1-yl)ethanimine N-oxide (1, $R^1$ = 4-Cl) with allyl 4-methoxyphenyl ether (2, $R^2$ = 4-$OCH_3$). The resulting cis-and trans-diastereomeric mixture of compound 3 ($R^1$ = 4-Cl, $R^2$ = 4-$OCH_3$) was flash-chromatographed on neutral silica gel using chloroform-methanol (98:2 by volume) as eluent. Isomer A has a melting point of 109-111°C (ethyl acetate-hexane, 1:1 by volume).
Anal. Calcd for $C_{21}H_{23}ClN_4O_3$: C, 60.79; H, 5.59; N, 13.50; Cl, 8.55. Found: C, 60.75; H, 5.69; N, 13.48; Cl, 8.59.



BAD ORIGINAL

Example 9

3-(4-Chlorophenyl)-3-(1H-1,2,4-triazol-1-ylmethyl)-2-methyl -5-[(4-chloro-3-methylphenyl)methyl]isoxazolidine (3, $R_1$=4-Cl, $R^2$ =4-Cl, 3-$CH_3$)

Compound 3 ($R^1$=4-Cl , $R^2$=4-Cl,3-$CH_3$) was prepared by a procedure similar to that described in Example 1 by reacting 1-(4-chlorophenyl)-N-methyl-2-(1H-1,2,4-triazol-1-yl)ethanimine N-oxide (1, $R^1$=4-Cl) with allyl 4-chloro-3-methylphenyl ether ( 2, $R^2$=4-Cl,3-$CH_3$). The resulting cis-and trans-diastereomeric mixture of the title compound was flash-chromatographed on neutral silica gel using chloroform-methanol (98:2 by volume) as eluent. Isomer A has a melting point of 135-139°C (ethyl-hexane, 1:1 by volume)
Anal. Calcd $C_{21}H_{22}Cl_2N_4O_2$: C, 58.21; H, 5.12; N, 12.93; Cl, 16.36. Found: C, 58.41; H, 5.29; N, 12.89; Cl, [illegible].71.

Example 10

3-(4-Chlorophenyl)-3-(1H-1,2,4-triazol-1-ylmethyl)-2-methyl -5-[(4-acetamidophenoxy)methyl]isoxazolidine (3, $R^1$=4-Cl, $R^2$=4-$NHCOCH_3$)

Compound 3 ($R^1$=4-Cl, $R^2$=4-$NHCOCH_3$) was prepared by a procedure similar to that described in Example 1 by reacting 1-(4-chlorophenyl)-N-methyl-2-(1H-1,2,4-triazol-1-yl)ethanimine N-oxide (1, $R^1$=4-Cl) with 4-acetamidophenyl allyl ether (2, $R^2$=4-$NHCOCH_3$).The resulting cis-and trans-diastereomeric mixture of compound 3 ($R^1$=4-Cl, $R^2$=4-$NHCOCH_3$) was flash-chromatographed on neutral silica gel using chloroform-methanol (99:1 by volume) as eluent. Isomer A has a melting point of 150-152°C (benzene).
Anal. Calcd for $C_{22}H_{24}ClN_5O_3$: C, 59.79; H, 5.47; N, 15.85; Cl, 8.02. Found: C, 59.68; H, 5.72; N, 15.44; Cl, 7.73.

Example 11

3-(4-Methoxyphenyl)-3-(1H-1,2,4-triazol-1-ylmethyl)-2-methyl-5-[(4-chlorophenoxy)methyl]isoxazolidine (3, $R^1$=4-$OCH_3$, $R^2$=4-Cl)

Compound 3 ($R^1$=4-$OCH_3$, $R^2$=4-Cl) was prepared by a procedure similar to that described in Example 1 by reacting 1-(4-methoxyphenyl)-N-methyl-2-(1H-1,2,4-triazol-1-yl)ethanimine N-oxide ( 1, $R^1$=4-$OCH_3$) with allyl 4-chlorophenyl ether (2 , $R^2$=4-Cl). The resulting cis-and trans-diastereomeric mixture of the title derivative was flashchromatographed on neutral silica gel using chloroform-methanol (98:2 by volume) as eluent. Isomer A has a melting point of 143-146°C (ethyl acetate).
Anal. Calcd for $C_{21}H_{23}ClN_4O_3$: C, 60.79; H, 5.59; N, 13.50; Cl, 8.55. Found: C, 60.92; H, 5.67; N, 13.56; Cl, 8.64.

Example 12

3-(4-Chlorophenyl)-3-(1H-1,2,4-triazol-1-ylmethyl)-2-methyl -5-{[4-(methoxycarbonyl)phenoxy]-methyl}isoxazolidine (3, $R^1$=4-Cl, $R^2$=4-$CO_2CH_3$)

Compound 3 ($R^1$=4-Cl, $R^2$=4-$CO_2CH_3$) was prepared by a procedure similar to that described in Example 1 by reacting 1-(4-chlorophenyl)-N-methyl-2-(1H-1,2,4-triazol-1-yl)-ethanimine N-oxide ( 1, $R^1$=4-Cl) with methyl 4-allyloxybenzoate (2, $R^2$=4-$CO_2CH_3$). The resulting cis-and trans-diastereomeric mixture of the title derivative was flash-chromatographed on neutral silica gel using chloroform-methanol (98:2 by volume) as eluent. Isomer A has a melting point of 153-156°C (ethyl acetate).
Anal. Calcd for $C_{22}H_{23}ClN_4O_4$: C, 59.66; H, 5.23; N, 12.65; Cl, 8.00. Found: C, 59.64; H, 5.32; N, 12.58; Cl, 8.09. Isomer B has a melting point of 145-148°C (ethyl acetate-hexane, 1:1 by volume).
Anal. Calcd for $C_{22}H_{23}ClN_4O_4$: C, 59.66; H, 5.23; N, 12.65; Cl, 8.00. Found: C, 59.59; H, 5.40; N, 12.53; Cl, 5.93.



BAD ORIGINAL

Example 13

3-(4-Chlorophenyl)-3-(1H-1,2,4-triazol-1-ylmethyl)-2-methyl-5-n-hexadecylisoxazolidine (5: $R^1$ = 4-Cl, n = 15)

A solution of 32.8 g (0.131 mol) of 1-(4-chlorophenyl)-N-methyl-2-(1H-1,2,4-triazol-1-yl)ethanimine N-oxide (1: $R^1$ = 4-Cl) [prepared by reacting 2-(1H-1,2,4-triazol-1-yl)-4'-chloroacetophenone (35.38 g, 0.160 mol) with N-methylhydroxylamine hydrochloride (20.0 g, 0.240 mol) and $NaHCO_3$ (20.12 g, 0.240 mol) in 500 ml ethanol] and 62.8 ml (0.196 mol) of 1-octadecene (4: n = 15) in 400 ml toluene is refluxed for 48 hours under a nitrogen atmosphere. Upon cooling to room temperature the solvent is removed under reduced pressure. The residual dark solid is collected and washed sequentially with acetonitrile and ether. Crystallization from ethyl acetate gave 9.0 g (14%) of isomer A (5: $R^1$ = 4-Cl, n = 15), melting at 100-102°C.

Anal. Calcd for $C_{29}H_{47}ClN_4O$: C, 69.22; H, 9.42; N, 11.13; Cl, 7.05. Found: C, 69.34; H, 9.33; N, 11.05; Cl, 7.39.

Example 14

3-Phenyl-3-(1H-1,2,4-triazol-1-ylmethyl)-2-methyl-5-n-hexylisoxazolidine (5: $R^1$ = H, n = 5)

Derivative 5 ($R^1$ = H, n = 5) is prepared by a procedure similar to that described in Example 13 by reacting 1-phenyl-N-methyl-2-(1H-1,2,4-triazol-1-yl)ethanimine N-oxide (1: $R^1$ = H) with 1-octene (4: n = 5). The resulting cis -and trans-diastereomeric mixture of compound 5 ($R^1$ = H, n = 5) is flash-chromatographed on neutral silica gel using as eluent a 98:2 by volume mixture of chloroform-methanol. Isomer A has a melting point of 93-99°C (ethyl acetate).

Anal. Calcd for $C_{19}H_{28}N_4O$: C, 69.48; H, 8.59; N, 17.06. Found: C, 69.45; H, 8.63; N, 17.08.

Example 15

3-(4-Methoxyphenyl)-3-(1H-1,2,4-triazol-1-ylmethyl)-2-methyl -5-n-octylisoxazolidine (5: $R^1$ = 4-$OCH_3$, n = 7)

Derivative 5 ($R^1$ = 4-$OCH_3$, n = 7) is prepared by a procedure similar to that described in Example 13 by reacting 1-(4-methoxyphenyl)-N-methyl-2-(1H-1,2,4-triazol-1-yl)ethanimine N-oxide (1: $R^1$ = 4-$OCH_3$) with 1-decene (4: n = 7). The resulting diastereomeric mixture of cis-and trans-derivative 5 ($R^1$ = 4-$OCH_3$), n = 7) is flash-chromatographed on neutral silica gel using chloroform-methanol (99:1 by volume) as eluent. Isomer A has a melting point of 89-91°C (ethyl acetate).

Anal. Calcd for $C_{22}H_{34}N_4O_2$: C, 68.36; H, 8.87; N, 14.49. Found C, 68.48; H, 8.76; N, 14.52.

Example 16

3-(4-Chlorophenyl)-3-(1H-1,2,4-triazol-1-ylmethyl)-2-methyl-5-n-decylisoxazolidine (5: $R^1$ = 4-Cl, n = 9)

Derivative 5 ($R^1$ = 4-Cl, n = 9) is prepared by a procedure similar to that described in Example 13 by reacting 1-(4-chlorophenyl)-N-methyl-2-(1H-1,2,4-triazol-1-yl)ethanimine N-oxide (1: $R^1$ = 4-Cl) with 1-dodecene (4: n = 9). The resulting diastereomeric mixture of cis-and trans-derivative 5 ($R^1$ = 4-Cl, n = 9) is purified by fractional crystallization using ethyl acetate as solvent. Isomer A has a melting point of 91-94°C (ethyl acetate).

Anal. Calcd for $C_{23}H_{35}ClN_4O$: C, 65.93; H, 8.42; N, 13.37; Cl, 8.46. Found C, 65.87; H, 8.28; N, 13.29; Cl, 8.79.

Derivative 5 where n = 18 is prepared by a procedure similar to that described in Example 13 by substituting 1-heneicosene for 1-octadecene.



BAD ORIGINAL

Example 17

3-(4-Methoxyphenyl)-5-(3-nitrophenyl)-2-methyl-3-(1H-1,2,4 -triazol-1-ylmethyl)isoxazolidine (7: $R^1$ = 4-$OCH_3$, $R^2$ 3-$NO_2$

A solution of 10.38 g (0.042 mol) of 1-(4-methoxyphenyl) -N-methyl-2-(1H-1,2,4-triazol-1-yl)ethanimine N-oxide (1 : $R^1$ = 4-$OCH_3$) [prepared by reacting 2-(1H-1,2,4-triazol-1-yl) -4'-methoxyacetophenone (32.15 g, 0.148 mol) with N-methylhydroxylamine hydrochloride (19.34 g, 0.232 mol) and $NaHCO_3$ (19.45 g, 0.232 mol) in 300 ml ethanol] and 8.81 ml (0.063 mol) of 3-nitrostyrene (6: $R^2$ = 3-$NO_2$) in 400 ml toluene is refluxed for 48 hours under a nitrogen atmosphere. Upon cooling to room temperature, the solvent is removed in vacuo, leaving a dark oil containing the cis-and trans-diastereomeric mixture of compound 7 ($R^1$ = 4-$OCH_3$, $R^2$ = 3-$NO_2$). Fractional crystallization from ether gave 6.92 g (42%) of isomer A, melting point 147-150°C (ethyl acetate).
Anal . Calcd. for $C_{20}H_{21}N_5O_4$: C, 60.75; H, 5.35; N, 17.71. Found: C. 60.61; H, 5.35; N, 17.52.

Example 18

3,5-Diphenyl-2-methyl-3-(1H-1,2,4-triazol-1-ylmethyl)-isoxazolidine (7: $R^1$ = $R^2$ = H)

Compound 7 ($R^1$ = $R^2$ = H) is prepared by a method similar to that described in Example 17 by reacting N-methyl-1-phenyl-2-(1H-1,2,4-triazol-1-yl)ethanimine N-oxide (1: $R^1$ = H) with styrene (6: $R^2$ = H). The resulting cis -and trans-diastereomeric mixture of compound 7 ($R^1$ = $R^2$ = H) is purified by fractional crystallization using ethyl acetate. Isomer A has a melting point of 96-102°C (ethyl acetate). Anal . Calcd. for $C_{19}H_{20}N_4O$: C, 71.23; H, 6.29; N, 17.49. Found C, 71.17; H, 6.48; N, 17.41.

Example 19

3-Phenyl-5-(4-fluorophenyl)-2-methyl-3-(1H-1,2,4-triazol-1-ylmethyl)isoxazolidine (7: $R^1$ = H, $R^2$ = 4-F)

Compound 7 ($R^1$ = H, $R^2$ = 4-F) is prepared by a method similar to that described in Example 17 by reacting N-methyl-1-phenyl-2-(1H-1,2,4-triazol-1-yl)ethanimine N-oxide (1: $R^1$ = H) with 4-fluorostyrene (6: $R^2$ = 4-F). The resulting cis-and trans-diastereomeric mixture of compound 7 ($R^1$ = H, $R^2$ = 4-F) is flash-chromatographed on neutral silica gel using a 98:2 by volume mixture of chloroform and methanol as eluent. Isomer A has a melting point of 105-109°C (ethyl acetate). Anal . Calcd. for $C_{19}H_{19}FN_4O$: C, 67.44; H, 5.66; N, 16.56; F, 5.61. Found: C, 67.43; H, 5.48; N, 16.64; F, 5.57.

Example 20

3-Phenyl-5-(4-methylphenyl)-2-methyl-3-(1H-1,2,4-triazol--1-ylmethyl)isoxazolidine (7: $R^1$ = H, $R^2$ = 4-$CH_3$)

Compound 7 ($R^1$ = H, $R^2$ = 4-$CH_3$) is prepared by a method similar to that described in Example 17 by reacting N-methyl-1-phenyl-2-(1H-1,2,4-triazol-1-yl)ethanimine N-oxide (1: $R^1$ = H) with 4-methylstyrene (6: $R^2$ = 4-$CH_3$). The resulting cis-and trans-diastereomeric mixture of compound 7 ($R^1$ = H, $R^2$ = 4-$CH_3$) is flash-chromatographed on neutral silica gel using 98:2 by volume mixture of chloroform and methanol as eluent. Isomer A has a melting point of 110-114°C (ethyl acetate).
Anal. Calcd. for $C_{20}H_{22}N_4O$: C, 71.83; H, 6.63; N, 16.75. Found: C, 71.64; H, 6.66; N, 16.67.



BAD ORIGINAL

Example 21

3-(4-Chlorophenyl)-5-phenyl-2-methyl-3-(1H-1,2,4-triazol-1-ylmethyl)-isoxazolidine (7: $R^1$ = 4-Cl, $R^2$ = H)

Compound 7 ($R^1$ = 4-Cl, $R^2$ = H) is prepared by a method similar to that described in Example 17 by reacting 1-(4-chlorophenyl)-N-methyl-2-(1H-1,2,4-triazol-1-yl)ethanimine N-oxide (1: $R^1$ = 4-Cl) with styrene (6: $R^2$ = H). The resulting cis-and trans-diastereomeric mixture of compound 7 ($R^1$ = 4-Cl, $R^2$ = H) is flash-chromatographed on neutral silica gel using a 99:1 by volume mixture of chloroform and ethanol as eluent.

Isomer A has a melting point of 128-131°C (ethyl acetate-hexane, 1:1 by volume). Anal. Calcd. for $C_{19}H_{19}ClN_4$): C, 64.31; H, 5.40; N, 15.79; Cl, 9.99. Found: C, 64.28; H, 5.42; N, 15.78; Cl, 10.11.

Isomer B has a melting point of 119-122°C (ethyl acetate-hexane, 1:1 by volume). Anal. Calcd. for $C_{19}H_{19}ClN_4O$: C, 64.31; H, 5.40; N, 15.79; Cl, 9.99. Found: C, 64.24; H, 5.49; N, 15.78; Cl, 10.08.

Example 22

3,5-Bis(4-chlorophenyl)-2-methyl-3-(1H-1,2,4-triazol-1-yl-methyl)isoxazolidine (7: $R^1$ = $R^2$ -4-Cl)

Compound (7: ($R^1$ = $R^2$ = 4-Cl) is prepared by a method similar to that described in Example 17 by reacting 1-(4-chlorophenyl)-N-methyl-2-(1H-1,2,4-triazol-1-yl)ethanimine N-oxide (1: $R^1$ = 4-Cl) with 4-chlorostyrene (6: $R^2$ = 4-Cl). The resulting cis-and trans-diastereomeric mixture of compound 7 ($R^1$ = $R^2$ = 4-Cl) is flash-chromatographed on neutral silica gel using a 98:2 by volume mixture of chloroform and methanol as eluent.

Isomer A has a melting point of 117-120°C (ethyl acetate-hexane, 1:1 by volume). Anal. Calcd. for $C_{19}H_{18}Cl_2N_4O$: C, 58.62; H, 4.66; N, 14.39; Cl, 18.21. Found: C, 58.65; H, 4.82; N, 14.21; Cl, 17.85.

Isomer B has a melting point of 144-147°C (ethyl acetate).Anal. Calcd. for $C_{19}H_{18}Cl_2N_4O$: C, 58.62; H, 4.66; N, 14.39; Cl, 18.21. Found: C, 58.51; H, 4.74; N, 14.33; Cl, 18.26.

Example 23

3-(4-Chlorophenyl)-5-(4-fluorophenyl)-2-methyl-3-(1H-1,2, 4-triazol-1-ylmethyl)isoxazolidine (7: $R^1$ = 4-Cl, $R^2$ = 4-F)

Compound 7 ($R^1$ = 4-Cl, $R^2$ = 4-F) is prepared by a method similar to that described in Example 17 by reacting 1-(4-chlorophenyl)-N-methyl-2-(1H-1,2,4-triazol-1-yl)ethanimine N-oxide (1: $R^1$ = 4-Cl) with 4-fluorostyrene (6: $R^2$ = 4-F). The resulting cis-and trans-diastereomeric mixture of compound 7 ($R^1$ = 4-Cl, $R^2$ = 4-F) is flash-chromatographed on neutral silica gel using a 98:2 by volume mixture of chloroform and methanol as eluent.

Isomer A has a melting point of 138-140°C (ethyl acetate-hexane, 1:1 by volume). Anal. Calcd. for $C_{19}H_{18}ClFN_4O$: C, 61.21; H, 4.87; N, 15.03; Cl, 9.51; F, 5.10. Found: C, 61.22; H, 4.78; N. 14.95; Cl, 9.63; F, 5.00.

Example 24

3-(4-Fluorophenyl)-5-phenyl-2-methyl-3-(1H-1,2,4-triazol-1-ylmethyl)isoxazolidine (7: $R^1$ = 4-F, $R^2$ = H)

Compound 7 ($R^1$ = 4-F, $R^2$ = H) is prepared by a method similar to that described in Example 17 by reacting 1-(4-fluorophenyl)-N-methyl-2-(1H-1,2,4-triazol-1-yl)ethanimine N-oxide (1: $R^1$ = 4-F) with styrene (6: $R^2$ = H). The resulting cis and trans-diastereomeric mixture of compound 7 ($R^1$ = 4-F, $R^2$ = H) is flash-chromatographed on neutral silica gel using a 98:2 by volume mixture of chloroform and methanol as eluent.

Isomer A has a melting point of 122-125°C (ethyl acetate). Anal. Calcd. for $C_{19}H_{19}FN_4O$: C, 67.44; H, 5.66; N, 16.56; F, 5.61. Found: C, 67.07; H, 5.59; N, 16.66; F. 5.55.



BAD ORIGINAL

Example 25

3,5-Bis(4-fluorophenyl)-2-methyl-3-(1H-1,2,4-triazol-1-ylmethyl)isoxazolidine (7: $R^1$ = $R^2$ = 4-F)

Compound 7 ($R^1$ = $R^2$ = 4-F) is prepared by a method similar to that described in Example 17 by reacting 1-(4-fluorophenyl)-N-methyl-2-(1H-1,2,4-triazol-1-yl)ethanimine N-oxide (1: $R^1$ = 4-F) with 4-fluorostyrene (6: $R^2$ = 4-F). The resulting cis-and trans-diastereomeric mixture of compound 7 ($R^1$ = $R^2$ = 4-F) is flash-chromatographed on neutral silica gel using a 98:2 by volume mixture of chloroform and methanol as eluent.

Isomer A has a melting point of 98-103°C (ethyl acetate-hexane, 1:1 by volume). Anal. Calcd. for $C_{19}H_{18}F_2N_4O$: C, 64.04; H, 5.09; N, 15.72; F, 10.66. Found: C, 63.93; H, 5.05, N, 15.68; F, 10.59.

Example 26

3-(4-Methoxyphenyl)-5-(3,4-dimethoxyphenyl)-2-methyl-3-(1H-1,2,4-triazol-1-ylmethyl)isoxazolidine [7: $R^1$ = 4-$OCH_3$, $R^2$ = 3,4-$(OCH_3)_2$]

Compound 7 [$R^1$ = 4-$OCH_3$, $R^2$ = 3,4-$(OCH_3)_2$] is prepared by a method similar to that described in Example 17 by reacting 1-(4-methoxyphenyl)-N-methyl-2-(1H-1,2,4-triazol-1-yl)ethanimine N-oxide ( 1: $R^1$ = 4-$OCH_3$) with 3,4-dimethoxystyrene [6: $R^2$ = 3,4-$(OCH_3)_2$]. The resulting cis-and trans -diastereomeric mixture of compound 7 ($R^1$ = 4-$OCH_3$), is purified by fractional crystallization using ethyl acetate.
Isomer A has a melting point of 177-179°C (ethyl acetate). Anal. Calcd. for $C_{22}H_{26}N_4O_4$: C, 64.38; H, 6.38; N, 13.65. Found: C, 64.01; H, 6.51; N, 13.60.

Other compounds of the invention where $R^1$ includes mono or disubstitution with halogen, lower alkyl and/or lower alkoxy are prepared starting with nitrones 1 formed from triazolylacetophenones such as:
2-(1H-1,2,4-triazol-1-yl)-4′-methylacetophenone 2-(1H-1,2,4-triazol-1-yl)-3′ ,4′-dichloroacetophenone, 2-(1H-1,2,4-triazol-1-yl)-4′-chloro-3′-methylacetophenone.
2-(1H-1,2,4-triazol-1-yl)-3′-methoxyacetophenone, 2-(1H-1,2,4-triazol-1-yl)-3′-methylacetophenone.

Compound 7 of the invention where $R^2$ includes trifluoromethyl can be prepared according to the method of Example 17 by substituting for 3-nitrostyrene,
3-(trifluoromethyl)styrene, bp 55°C/17 mm.

Salts of the compounds of the invention can be prepared as known in the art, for example, by dissolving the compound in a 10:1 by volume mixture of ethanol and aqueous acid such as HCl or $HNO_3$, evaporating the solvent, and then recrystallizing the crude salt, for example, from methanolether, 1:3 by volume in the case of HCl salts, and ethanol in the case of $HNO_3$ salts.

## Claims

1. A compound of the formula:

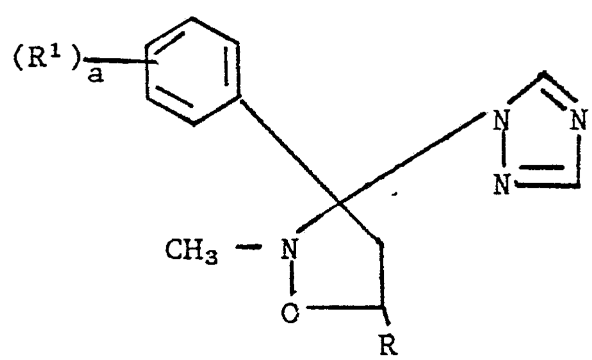

wherein;
a = 1 or 2
$R^1$ is selected from hydrogen, lower alkyl, lower alkoxy, halogen and combinations thereof provided that the



BAD ORIGINAL

ortho position is hydrogen, and
R is selected from.

(a) 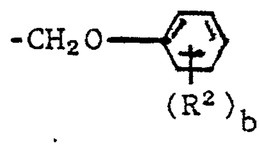

wherein;
b = 1 or 2, and
$R^2$ is selected from hydrogen, lower alkyl, lower alkoxy, halogen, alkoxycarbonyl, alkanoylamino, nitro, and combinations thereof,
(b) $-(CH_2)_nCH_3$
wherein n = 1 to 18, branched or unbranched chain, and

(c) 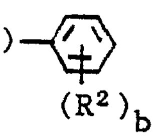

wherein,
b = 1 or 2, and
$R^2$ is selected from hydrogen, lower alkyl, lower alkoxy, halogen, trifluoromethyl, nitro, and combinations thereof,
and pharmaceutically acceptable acid addition salts thereof, in the form of their enantiomers, or mixtures of their enantiomers including diastereoisomeric pairs of such enantiomers.

2. A compound according to Claim 1 wherein R is:

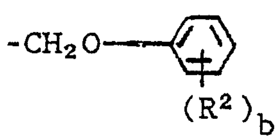

wherein;
b = 1 or 2, and
$R^2$ is selected from hydrogen, lower alkyl, lower alkoxy, halogen, alkoxycarbonyl, alkanoylamino, nitro, and combinations thereof.

3. A compound according to Claim 1 wherein R is:
$-(CH_2)_n CH_3$
wherein, n = 1 to 18 branched or unbranched chain.

4. A compound according to Claim 1 wherein R is:

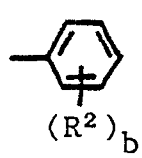

wherein;
b = 1 or 2, and
$R^2$ is selected from hydrogen, lower alkyl, lower alkoxy, halogen, trifluoromethyl, nitro, and combinations thereof.

5. The compound of claim 2 wherein the compound is 3-(4-chlorophenyl)-3-(1H-1,2,4-triazol-1-ylmethyl)-2-methyl -5-[(4-chlorophenoxy)methyl]isoxazolidine.



BAD ORIGINAL

6. The compound of claim 2 where in the compound is 3-(4-chlorophenyl)-3-(1H-1,2,4-triazol-1-ylmethyl)-2-methyl-5-[(methoxyphenoxy)methyl]isoxazolidine.

7. The compound of claim 2 wherein the compound is 3-(4-chlorophenyl)-3-(1H-1,2,4-triazol-1-ylmethyl)-2-methyl-5-[(4-chloro-3-methylphenyl)methyl]isoxazolidine.

8. The compound of claim 2 wherein the compound is 3-(4-methoxyphenyl)-3-(1H-1,2,4-triazol-1-ylmethyl)-2-methyl-5-[(4-chlorophenoxy)methyl]isoxazolidine.

9. The compound of claim 4 wherein the compound is 3,5-bis(4-chlorophenyl)-2-methyl-3-(1H-1,2,4-triazol-1-ylmethyl)isoxazolidine.

10. The compound of claim 4 wherein the compound is 3-(4-chlorophenyl)-5-(4-fluorophenyl)-2-methyl-3-(1H-1,2,4-triazol-1-ylmethyl)isoxazolidine.

11. The compound of Claim 4 wherein the compound is 3,5-bis(4-fluorophenyl)-2-methyl-3-(1H-1,2,4-triazol-1-ylmethyl)isoxazolidine.

Claims for the following contracting states: AT and ES

1. A process for preparing a compound having the formula:

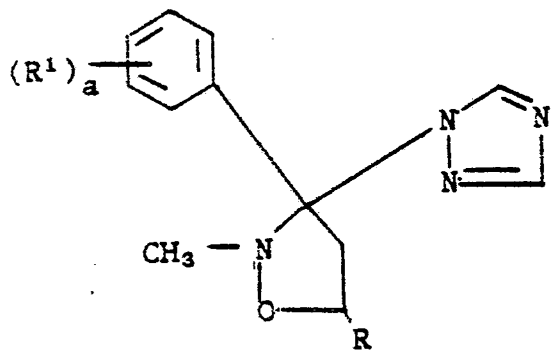

wherein
a = 1 or 2,
$R^1$ is selected from hydrogen, lower alkyl, lower alkoxy, halogen and combinations thereof provided that the ortho position is hydrogen, and
R is selected from,

(a) 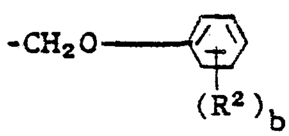

wherein
b = 1 or 2, and
$R^2$ is selected from hydrogen, lower alkyl, lower alkoxy, halogen, alkoxycarbonyl, alkanoylamino, nitro, and combinations thereof,
(b) $-(CH_2)_nCH_3$
wherein n = 1 to 18, branched or unbranched chain, and

(c) 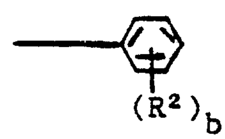

wherein
b = 1 or 2, and
$R^2$ is selected from hydrogen, lower alkyl, lower alkoxy, halogen, trifluoromethyl, nitro, and combinations thereof, and pharmaceutically acceptable acid addition salts thereof,



BAD ORIGINAL

in the form of their enantiomers, or mixtures of their enantiomers including disastereoisomeric pairs of such enantiomers, comprising reacting a nitrone of the formula;

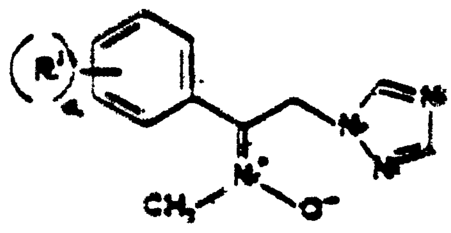

in which $(R^1)_a$ is as defined above,

(1) when R is (a) above, with an allyl phenyl ether of the formula

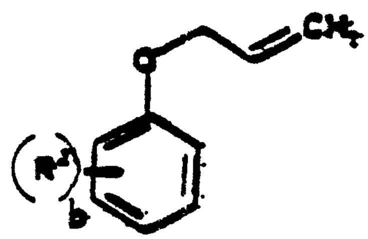

in which $(R^2)_b$ is as defined in (a) above,

(2) when R is (b) above, with a 1-alkene having 4 to 21 carbons of the formula:

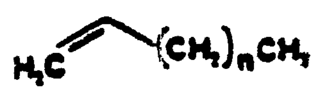

where n = 1 to 18, and

(3) when R is (c) above, with a styrene of the formula:

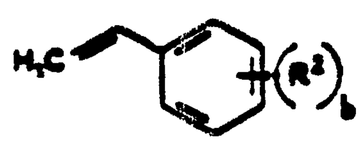

in which $(R^2)_b$ is as defined in (c) above, and optionally converting the resulting compound into its pharmaceutically acceptable acid addition salt.

2. The process of claim 1 wherein the compound is reacted with an acid to form a pharmaceutically acceptable acid addition salt of the compound.

3. The process claim 1 wherein 3-(4-chlorophenyl)-3-(1H-1,2,4-triazole-1-ylmethyl)-2-methyl-5-[(4-chlorophenoxy)methyl]isoxazolidine is prepared by reacting 1-(4-chlorophenyl)-N-methyl-2-(1H-1,2,4-triazole-1-yl)ethanimine N-oxide with allyl 4-chlorophenyl ether.

4. The process of claim 1 wherein 3-(4-chlorophenyl) -3-(1H-1,2,4-triazole-1-ylmethyl)-2-methyl-5-[(4-methoxyphenoxy)methyl]isoxazolidine is prepared by reacting 1-(4-chlorophenyl)-N-methyl-2-(1H-1,2,4-triazol-1-yl)ethanimine N-oxide with allyl 4-methoxyphenyl ether.

5. The process of claim 1 wherein 3-(4-chlorophenyl) -3-(1H-1,2,4-triazol-1-ylmethyl)-2-methyl-5-[(4-chloro-3-methylphenyl)methyl]isoxazolidine is prepared by reacting 1-(4-chlorophenyl)-N-methyl-2-(1H-1,2,4-triazol-1-yl)ethanimine N-oxide with allyl 4-chloro-3-methylphenyl ether.

6. The process of claim 1 wherein 3-(4-methoxyphenyl)-3-(1H-1,2,4-triazol-1-ylmethyl)-2-methyl-5-[(4-chlorophenoxy)methyl]isoxazolidine is prepared by reacting 1-(4-methoxyphenyl)-N-methyl-2-(1H-1,2,4-triazole -1-yl)ethanimine N-0xide with allyl 4-chlorophenyl ether.

7. The process of claim 1 wherein 3,5-bis(4-chlorophenyl)-2-methyl-3-(1H-1,2,4-triazol-1-yl-methyl)-isoxazolidine is prepared by reacting 1-(4-chlorophenyl)-N-methyl-2-(1H-1,2,4-triazol-1-yl)ethamine N-oxide with 4-chlorostyrene.

8. The process of claim 1 wherein 3-(4-chlorophenyl)-5-(4-(fluorophenyl)-2-methyl-3-(1H-1,2,4-triazole-1-ylmethyl))isoxazolidine is prepared by reacting 1-(4-chlorophenyl)-N-methyl-2-(1H-1,2,4-triazole-1-yl)-ethanimine N-oxide with 4-fluorostyrene.

9. The process of claim 1 wherein 3,5-bis(4-fluorophenyl)-2-methyl-3-(1H-1,2,4-triazole-1-ylmethyl)-isoxazolidine is prepared by reacting 1-(4-fluorophenyl)-N-methyl-2-(1H-1,2,4-triazol-1-yl)ethanimine N-oxide with 4-fluorostyrene.